# NEW EUROPEAN PATENT SPECIFICATION

(11) **EP 1 237 948 B2**
(45) Date of publication and mention of the opposition decision: **07.03.2007**
(45) Mention of the grant of the patent: 07.01.2004
(21) Application number: 00967007.6
(22) Date of filing: 28.09.2000
(51) Int. Cl.: C08F 10/00, C08F 4/602

(54) **PREPARATION OF CURABLE POLYMERS**
HERSTELLUNG VON HÄRTBAREN POLYMEREN
PREPARATION DE POLYMERES DURCISSABLES

(30) Priority: 29.09.1999 US 156550 P
(43) Date of publication of application: 11.09.2002
(73) Proprietor: E.I. DU PONT DE NEMOURS AND COMPANY, Wilmington, Delaware 19898 (US)
(72) Inventor: WANG, Lin, Hockessin, DE 19707 (US); CITRON, Joel, David, Wilmington, DE 19810 (US)
(74) Representative: Towler, Philip Dean
(86) International application number: PCT/US2000/026647
(87) International publication number: WO 2001/023445

(56) References cited:
- EP-B- 1 237 948
- WO-A-00/55216
- WO-A-97/48735
- WO-A-99/46302
- WO-A-99/50318
- MECKING S: "REACTOR BLENDING WITH EARLY/LATE TRANSITION METAL CATALYST COMBINATIONS IN ETHYLENE POLYMERIZATION" MACROMOLECULAR: RAPID COMMUNICATIONS,DE,WILEY VCH, WEINHEIM, vol. 20, no. 3, March 1999 (1999-03), pages 139-143, XP000834998 ISSN: 1022-1336

## Description

### FIELD OF THE INVENTION

Olefin containing branched polyolefins, especially elastomers, are produced in processes using at least two active catalysts, one of which is a selected iron catalyst that oligomerizes ethylene, and another of which is a transition metal catalyst that copolymerizes ethylene, α-olefins and non-conjugated dienes.

### TECHNICAL BACKGROUND

Polyolefins containing olefinic unsaturation, especially elastomers, are particularly useful, since they may be cured (crosslinked/vulcanized) by the use of sulfur cures or free radical cures. Of particular interest are so-called EPDM elastomers, copolymers of ethylene, propylene and a nonconjugated diene such as ethylidene norbornene, dicyclopentadiene, or 1,4-hexadiene. However manufacture of EPDMs to produce good quality polymers may be difficult, since the correct proportions of ethylene and propylene must be in the polymers (and in the polymerization reactors) in a nonblocky manner to produce good elastomeric properties. Therefore, improved methods of producing (elastomeric) polyolefins which contain olefinic groups, and/or improved polymers with EPDM-like properties are desired.

EPDMs have been made using metallocene catalysts, see for instance US5229478, WO88/04674, WO99/18135 and WO99/01460, and references described therein.

Various reports of "simultaneous" oligomerization and polymerization of ethylene to form (in most cases) branched polyethylenes have appeared in the literature, see for instance WO90/15085, WO99/50318, US5753785, US5856610, US5686542, US5137994 and US5071927; C. Denger, et al, Makromol. Chem. Rapid Commun., vol. 12, p. 697-701 (1991); and E. A. Benham, et al., Polymer Engineering and Science, vol. 28, p. 1469-1472 (1988). None of these references specifically describes any of the processes or resulting branched polyolefins herein.

### SUMMARY OF THE INVENTION

This invention concerns a process for preparing a branched polyolefin containing olefinic bonds, comprising the steps of:
(1) contacting an ethylene oligomerization catalyst and a first monomer component comprising ethylene, under conditions to oligomerize at least a portion of the ethylene to one or more α-olefins, wherein the ethylene oligomerization catalyst comprises an active Fe complex of a ligand of the formula (I): 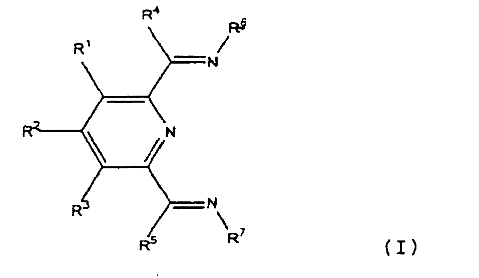 wherein:
   R¹, R², R³, R⁴ and R⁵ are each independently hydrogen, hydrocarbyl, substituted hydrocarbyl, or an inert functional group, provided that any two of R¹, R² and R³ vicinal to one another, taken together may form a ring; and
   R⁶ and R⁷ are aryl or substituted aryl; and
(2) contacting an active transition metal copolymerization catalyst, with a second monomer component comprising ethylene, at least a portion of the α-olefins from step (1) and an active nonconjugated diene, under conditions to copolymerize the second monomer component to a branched polyolefin containing olefinic bonds.

The two steps of the above-mentioned process may occur separately, sequentially and/or simultaneously, as described in further detail below.

The process as described above is capable of producing some novel branched polyolefins. One such novel branched polyolefin in accordance with the present invention contains at least 2 ethyl branches, at least 2 hexyl or longer branches and at least one butyl branch per 1000 methylene groups, and olefinic bonds. Another such novel branched polyolefin in accordance with the present invention contains 20 to 150 branches of the formula -(CH₂CH₂)ₙH, wherein n is an integer of 1 to 100, and olefinic bonds.

### DETAILED DESCRIPTION OF THE PREFERRED EMBODIMENTS

Herein certain terms are used which are defined below.

By "hydrocarbyl" is meant a univalent radical containing only carbon and hydrogen. As examples of hydrocarbyls may be mentioned unsubstituted alkyls, cycloalkyls and aryls. If not otherwise stated, it is preferred that the hydrocarbyl groups herein contain 1 to 30 carbon atoms, and more preferably 1 to 20 carbon atoms.

By "substituted hydrocarbyl" herein is meant a hydrocarbyl group that contains one or more "inert functional groups" that are inert under the process conditions to which the compound containing these groups is subjected. The inert functional groups also do not substantially interfere with the oligomerization/polymerization process. For example, in cases in which the inert functional group may be near the complexed iron atom, such as R⁴ or R⁵ in (I), or as a substituent on R⁴, R⁵, R⁶ or R⁷, the inert functional group should not coordinate to the iron atom more strongly than the three depicted N groups in (I) which are the desired coordinating groups - that is, the functional group should not displace one or more of the desired coordinating N groups. The hydrocarbyl may be completely substituted, as in trifluoromethyl. If not otherwise stated, it is preferred that substituted hydrocarbyl groups herein contain 1 to about 30 carbon atoms. Included in the meaning of "substituted" are heterocyclic rings.

Examples of inert functional groups include halo (fluoro, chloro, bromo and lodo), ester, keto (oxo), amino, imino, carboxyl, phosphite, phosphonite, phosphine, phosphinite, thioether, amide, nitrile, and ether. Preferred inert functional groups are halo, ester, amino, imino, carboxyl, phosphite, phosphonite, phosphine, phosphinite, thioether, and amide. Which inert functional groups are useful in which oligomerizations/polymerizations may in some cases be determined by reference to US5955555, US6103946 and WO98/30612.

By an oligomerization or polymerization "catalyst activator" is meant a compound that reacts with a transition metal compound to form an activated catalyst species. A preferred catalyst activator is an alkylaluminum compound, that is, a compound which has at least one alkyl group bound to an aluminum atom.

By "relatively noncoordinating" (or "weakly coordinating") anions are meant those anions as are generally referred to in the art in this manner, and the coordinating ability of such anions is known and has been discussed in the literature. See, for instance, W. Beck et al., Chem. Rev., vol. 88, pp. 1405-1421 (1988), and S.H. Strauss, Chem. Rev., vol. 93, pp. 927-942 (1993).

Among such anions are those formed from aluminum compounds (such as those described in the immediately. preceding paragraph) and X⁻ (an anion as discussed in further detail below), including (R¹⁹)₃AlX⁻, (R¹⁹)₂AlClX⁻, R¹⁹AlCl₂X⁻, and R¹⁹AlOX⁻, wherein R¹⁹ is alkyl. Other useful noncoordinating anions include BAF- {BAF = tetrakis [3,5-bis(trifluoromethyl)phenyl]borate}, SbF₆⁻, PF₆⁻, and BF₄⁻, trifluoromethanesulfonate, p-toluenesulfonate, (R_{f}SO₂)₂N⁻, and (C₆F₆)₄B⁻.

By an "active nonconjugated diene" is meant a diene that may be polymerized through one of the double bonds in the diene, while the other double bond is essentially inert under the polymerization conditions. This yields a repeat unit in the polymer that contains an olefin moiety in a branch which is part of that repeat unit. Suitable non-conjugated dienes have as the reactive olefinic bond a terminal olefin, as in 1,4-hexadiene, or a particularly strained olefin as the ring double bond in ethylidene norbornene. The inert double bond is generally an internal double bond, such as the double bond in the 4 position of 1,4-hexadiene or the exo double bond in ethylidene norbornene. Generally speaking nonconjugated olefins suitable for making EPDM elastomers are also suitable herein. Preferred nonconjugated dienes are 1,4-hexadiene, ethylidene norbornene and dicyclopentadiene, and ethylidene norbornene is more preferred.

By "contains olefinic bonds not associated with end groups" is meant that the polymer contains nonaromatic carbon-carbon double bonds that are not at the ends of chains. Preferably these olefinic bonds are in repeat units derived from nonconjugated diene monomers, as described herein. It is preferred that the branched polyolefins in accordance with the present invention contain at least some olefinic bonds not associated with end groups.

By a "primary carbon group" herein is meant a group of the formula -CH₂---, wherein the free valence --- is to any other atom, and the bond represented by the solid line is to a ring atom of an aryl or substituted aryl to which the primary carbon group is attached. Thus the free valence -- may be bonded to a hydrogen atom, a halogen atom, a carbon atom, an oxygen atom, a sulfur atom, etc. In other words, the free valence --- may be to hydrogen, hydrocarbyl, substituted hydrocarbyl or a functional group. Examples of primary carbon groups include -CH₃, -CH₂CH(CH₃)₂, -CH₂Cl, - CH₂C₆H₅, -OCH₃ and -CH₂OCH₃.

By a secondary carbon group is meant the group 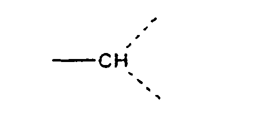 wherein the bond represented by the solid line is to a ring atom of an aryl or substituted aryl to which the secondary carbon group is attached, and both free bonds represented by the dashed lines are to an atom or atoms other than hydrogen. These atoms or groups may be the same or different. In other words the free valences represented by the dashed lines may be hydrocarbyl, substituted hydrocarbyl or inert functional groups. Examples of secondary carbon groups include -CH(CH₃)₂, -CHCl₂, -CH(C₆H₅)₂, cyclohexyl, - CH(CH₃)OCH₃, and -CH=CCH₃.

By a "tertiary carbon group" is meant a group of the formula 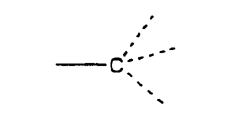 wherein the bond represented by the solid line is to a ring atom of an aryl or substituted aryl to which the tertiary carbon group is attached, and the three free bonds represented by the dashed lines are to an atom or atoms other than hydrogen. In other words, the bonds represented by the dashed lines are to hydrocarbyl, substituted hydrocarbyl or inert functional groups. Examples of tetiary carbon groups include -C(CH₃)₃, -C(C₆H₅)₃, -CCl₃, -CF₃, - C(CH₃)₂OCH₃, -C≡CH, -C(CH₃)₂CH=CH₂, aryl and substituted aryl such as phenyl and 1-adamantyl.

By "aryl" is meant a monovalent aromatic group in which the free valence is to the carbon atom of an aromatic ring. An aryl may have one or more aromatic rings which may be fused, connected by single bonds or other groups.

By "substituted aryl" is meant a monovalent aromatic group substituted as set forth in the above definition of "substituted hydrocarbyl". Similar to an aryl, a substituted aryl may have one or more aromatic rings which may be fused, connected by single bonds or other groups; however, when the substituted aryl has a heteroaromatic ring, the free valence in the substituted aryl group can be to a heteroatom (such as nitrogen) of the heteroaromatic ring instead of a carbon.

For ligand (I), preferred formulas and compounds (and for their Fe complexes also) are found in previously incorporated US6103946, and preferred groupings and compounds in this application are also preferred herein.

More specifically, the preferrred oligomerization catalyst is an Fe complex (Fe[II] or Fe[III]) of a ligand of the general formula (I), wherein:
R¹, R² and R³ are each independently hydrogen, hydrocarbyl, substituted hydrocarbyl or an inert functional group, provided that any two of R¹, R² and R³ vicinal to one another taken together may form a ring;
R⁴ and R⁵ are each independently hydrogen, hydrocarbyl, substituted hydrocarbyl or an inert functional group;
R⁶ and R⁷ are each independently an aryl or substituted aryl having a first ring atom bound to the imino nitrogen, provided that:
   in R⁶, a second ring atom adjacent to said first ring atom is bound to a halogen, a primary carbon group, a secondary carbon group or a tertiary carbon group; and further provided that
   in R⁶, when said second ring atom is bound to a halogen or a primary carbon group, none, one or two of the other ring atoms in R⁶ and R⁷ adjacent to said first ring atom are bound to a halogen or a primary carbon group, with the remainder of the ring atoms adjacent to said first ring atom being bound to a hydrogen atom; or
   in R⁶, when said second ring atom is bound to a secondary carbon group, none, one or two of the other ring atoms in R⁶ and R⁷ adjacent to said first ring atom are bound to a halogen, a primary carbon group or a secondary carbon group, with the remainder of the ring atoms adjacent to said first ring atom being bound to a hydrogen atom; or
   in R⁶, when said second ring atom is bound to a tertiary carbon group, none or one of the other ring atoms in R⁶ and R⁷ adjacent to said first ring atom are bound to a tertiary carbon group, with the remainder of the ring
   atoms adjacent to said first ring atom being bound to a hydrogen atom.

By a "first ring atom in R⁶ and R⁷ bound to an imino nitrogen atom" is meant the ring atom in these groups bound to an imino nitrogen shown in (I), for example 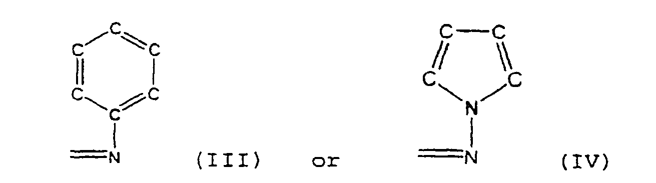 the atoms shown in the 1-position in the rings in (III) and (IV) are the first ring atoms bound to an imino carbon atom (other groups which may be substituted on the aryl groups are not shown). Ring atoms adjacent to the first ring atoms are shown, for example, in (V) and (VI), where the open valencies to these adjacent atoms are shown by dashed lines (the 2,6-positions in (V) and the 2,5-positions in (VI)).

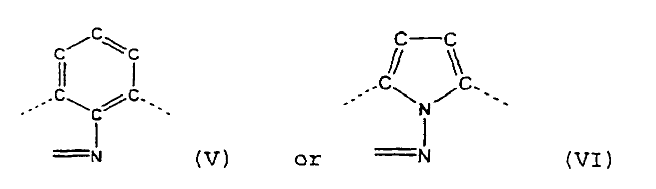

Particularly preferred is a ligand of the formula (II): 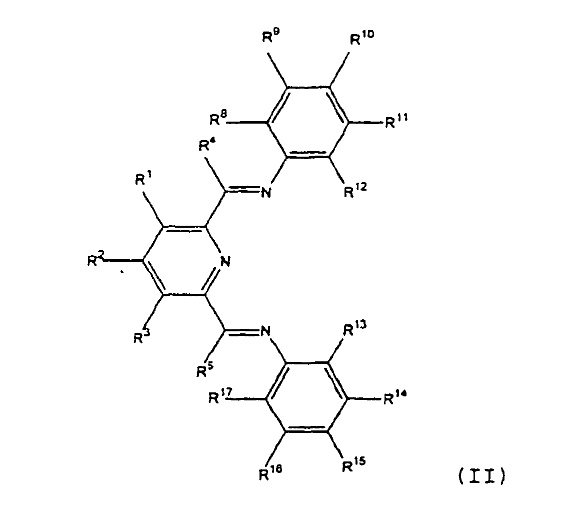 wherein:
each of R¹, R², R³, R⁴, R⁵, R⁹, R¹⁰, R¹¹, R¹⁴, R¹⁵ and R¹⁶ is independently selected from the group consisting of hydrogen, hydrocarbyl, substituted hydrocarbyl and an inert functional group; and
R⁸ is halogen, a primary carbon group, a secondary carbon group or a tertiary carbon group;
provided that:
when R⁸ is halogen or a primary carbon group none, one or two of R¹², R¹³ and R¹⁷ are independently a primary carbon group, an inert functional group or a trihalo tertiary carbon group, and the remainder of R¹², R¹³ and R¹⁷ are hydrogen;
when R⁸ is a secondary carbon group, none or one of R¹², R¹³ and R¹⁷ is a primary carbon group, a secondary carbon group, a trihalo tertiary carbon group or an inert functional group, and the remainder of R¹², R¹³ and R¹⁷ are hydrogen;
when R⁸ is a tertiary carbon group all of R¹², R¹³ and R¹⁷ are hydrogen;
any two of R¹, R² and R³ vicinal to one another, taken together may form a ring; and
any two of R⁸, R⁹, R¹⁰, R¹¹, R¹², R¹³, R¹⁴, R¹⁵, R¹⁶ and R¹⁷ vicinal to one another, taken together may form a ring.

In one preferred embodiment of ligand (II), R⁴ and R⁵ are methyl or hydrogen; and/or R¹, R², and R³ are all hydrogen; and/or R⁹, R¹⁰, R¹¹, R¹⁴ , R¹⁵ and R¹⁶ are all hydrogen; and/or R¹⁷ is selected from the group consisting of methyl, ethyl, propyl isopropyl, halo and trihalomethyl; and/or R¹² is selected from the group consisting of hydrogen, methyl, ethyl, propyl, isopropyl, halo and trihalomethyl. In certain more preferred embodiments, both R¹² and R¹⁷ are methyl or ethyl. In all such cases, R⁸ is a primary carbon group, and R¹³ is hydrogen.

In specific preferred embodiments of ligand (II) :
R⁴ and R⁵ are methyl; R⁹, R¹⁰, R¹¹, R¹³, R¹⁴, R¹⁵ and R¹⁶ are all hydrogen; R¹² is hydrogen or methyl; R¹⁷ is methyl; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydrogen or ethyl; R¹⁷ is ethyl; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydrogen or isopropyl; R¹⁷ is isopropyl; and R⁸ is a primary carbon group; or
R⁴ and R⁵ are methyl; R⁹, R¹⁰, R¹¹, R¹³, R¹⁴, R¹⁵ and R¹⁶ are all hydrogen R¹² is hydrogen or n-propyl; R¹⁷ is n-propyl; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydrogen or chloro; R¹⁷ is chloro; and R⁸ is a primary carbon group; or
R⁴ and R⁵ are methyl; R⁹, R¹⁰, R¹¹, R¹³, R¹⁴, R¹⁵ and R¹⁶ are all hydrogen; R¹² is hydorgen or trifluoromethyl;
R¹⁷ is trifluoromethyl; and R⁸ is a primary carbon group.

In another preferred embodiment of ligand (II), R¹, R², R³, R⁴, R⁵, R⁹, R¹⁰, R¹¹, R¹⁴, R¹⁵ and R¹⁶ are as just defined, and if R⁸ is a primary carbon group, R¹² and R¹⁷ are hydrogen, and R¹³ is a primary carbon group; or if R⁸ is a secondary carbon group, R¹² and R¹⁷ are hydrogen, and R¹³ is a primary carbon group or a secondary carbon group.

Also preferred is when R⁸ is a primary carbon group, preferably selected from methyl, ethyl, propyls and butyls.

Previously incorporated US5955555, US6103946 and WO98/30612, as well as WO99/50273 (equivalent to United States Patent Application Serial No. 08/277910, filed March 29, 1999) describe synthesis of ligand (I) and its Fe complexes, and reference may be had thereto for further details.

"Pure" Fe complexes may be exemplified by the formula (I)FeXₙ, wherein each X is an anion, n is 1, 2 or 3 so that the total number of negative charges on the X groups is equal to the oxidation state of the Fe in the pure Fe complex. Preferably, each X is a monovalent anion, more preferably selected from the group consisting of a halide and a nitrile, and especially a halide such as chloride or bromide.

These pure Fe complexes may in and of themselves be active oligomerization catalysts, or they may be activated (or made more active) preferably by preparation in situ by contact with a catalyst activator in a variety of methods. Generally, it has been found that the most active catalysts are those that have been contacted with a catalyst activator.

In general, details for the preparation of oligomers (sometimes referred to as α-olefins) from ethylene using the oligomerization catalysts herein can be found in previously incorporated us6103946, as well as B. L. Small, et. al., J. Am. Chem. Soc. , vol. 120, p. 7143-7144 (1998).

Ethylene may be oligomerized by contacting a first compound W, which is a neutral Lewis acid capable of abstracting X⁻ to form WX⁻, with an iron halide complex of ligand (I) (or other X⁻ complex of (I)), provided that the anion formed is a weakly coordinating anion; or a cationic Lewis or Bronsted acid whose counterion is a weakly coordinating anion.

In those instances in which the Fe complex of (I) does not contain an alkyl, hydride, or other group which may be displaced by ethylene already bonded to the metal (i.e., X is not alkyl or hydride), a neutral Lewis acid or a cationic Lewis or Bronsted acid may also alkylate or add a hydride to the metal, i.e., causes an alkyl group or hydride to become bonded to the metal atom, or a separate compound is added to add the alkyl or hydride group.

A preferred neutral Lewis acid, which can alkylate the metal, is a selected alkyl aluminum compound, such as R²⁰₃Al, R²⁰₃AlCl, R²⁰AlCl₂, and "R²⁰AlO" (alkylaluminoxanes), wherein R²⁰ is alkyl containing 1 to 25 carbon atoms, preferably 1 to 4 carbon atoms. Suitable alkyl aluminum compounds include methylaluminoxane (which is an oligomer with the general formula [MeAlO]ₙ), (C₂H₅)₂AlCl, (C₂H₅)AlCl₂ and [(CH₃)₂CHCH₂]₃Al. Metal hydrides such as NaBH₄ may be used to bond hydride groups to the metal M.

Preferably the oligomer produced by the oligomerization catalyst is a compound of the formula H₂C=CHR¹⁸,
wherein R¹⁸ is n-alkyl containing an even number of carbon atoms. Normally, the product of the oligomerization will be a mixture of oligomers of the above formula, preferably possessing a number average molecular weight of about 600 or less, more preferably about 400 or less. Other olefins, such as propylene may optionally be added to the process at any point, so that they also copolymerize into the polyolefin ultimately formed. Preferably, however, the only two monomers added to the system are ethylene the active nonconjugated diene (although, of course, other monomers are generated in situ from the oligomerization step).

The copolymerization catalyst is a catalyst chemically different from the oligomerization catalyst, and which is capable of copolymerizing ethylene, olefins of the formula H₂C=CHR¹⁸ (α-olefins) and active nonconjugated dienes, such as any one or combination of a number of well-known Ziegler-Natta-type or metallocene-type catalysts.

In polymerizations with the copolymerization catalysts alone, the resulting polymer tends to be relatively high molecular weight and uniform. The synthesis of the branched copolymers in accordance with the present invention herein can produce unique polymers because of the nature of the two catalysts. In preferred embodiments (discussed below) the oligomerization and copolymerization are performed simultaneously, and/or the oligomerization and copolymerization occur at comparable rates, to prepare various unique copolymers.

In one preferred form the process is carried out in the gas phase (although production of an elastomer may be difficult because of sticking of particles). It is believed that in many cases in gas phase polymerization when both catalysts are present in the same particle on which polymerization is taking place (for example originally a supported catalyst), the α-olefin is especially efficiently used (polymerized into the resulting polymer). The process may also be carried out in liquid slurry or solution.

The polymer produced usually contains only branches of the formula (excluding end groups and repeat units derived from the nonconjugated diene and olefins containing an odd number of carbon atoms) -(CH₂CH₂)ₙH wherein n is 1 or more, preferably 1 to 100, more preferably 1 to 30, of these branches per 1000 methylene groups. Normally there will be branches with a range of "n" in the polymer. The amount of these branches (as measured by total methyl groups) in the polymer preferably ranges from about 2 to about 200, especially preferably about 5 to about 175, more preferably about 10 to about 150, and especially preferably about 20 to about 150 branches per 1000 methylene groups in the polymer (for the method of measurement and calculation, US5880241, incorporated by reference herein). Another preferable range for these branches is about 50 to about 200 methyl groups per 1000 methylene carbon atoms. It is also preferable (either alone or in combination with the other preferable features above) that in these branched polymers there is at least 2 branches each of ethyl and n-hexyl or longer and at least one n-butyl per 1000 methylene groups, more preferably at least 4 branches each of ethyl and n-hexyl or longer and at least 2 n-butyl branches per 1000 methylene groups, and especially preferably at least 10 branches each of ethyl and n-hexyl or longer and at least 5 n-butyl branches per 1000 methylene groups. It is also preferred that there are more ethyl branches than butyl branches. In another preferred polymer (alone or in combination with any of the above preferred features) there is less than 20 methyl branches, more preferably less than 2 methyl branches, and especially preferably less than 2 methyl branches (all after correction for end groups) per 1000 methylene groups.

The product polymer preferably contains 0.1 to 10 percent, more preferably 0.5 to 8 percent, and especially preferably 1 to 5 percent by weight of repeat units derived from the nonconjugated diene. The amount of these units is based on the total weight of the polymer (all incorporated monomers). It may be determined by a number of suitable methods (with appropriate calibration) including IR spectroscopy, free olefin determination (as by bromine number), or ¹H or ¹³C NMR.

Conditions for such polymerizations, particularly for the oligomerization catalyst, are found in previously incorporated US6103946. Briefly, the temperature at which the polymerization is carried out is about -100°C to about +200°C preferably about -20°C to about +80°C. The polymerization pressure which is used with ethylene is not critical, atmospheric pressure to about 275 MPa, or more, being a suitable range. When run in a liquid, the nonconjugated diene may be used neat or (preferably) diluted with another liquid (solvent) for the monomer. These polymerizations may be batch, semi-batch or continuous processes, and may be carried out in liquid medium or the gas phase (assuming the diene has the requisite volatility).

The copolymerization catalyst may be a so-called Ziegler-Natta and/or metallocene-type catalyst. These types of catalysts are well known in the polyolefin field, see for instance Angew. Chem., Int. Ed. Engl., vol. 34, p. 1143-1170 (1995), EP-A-0416815 and US5198401 for information about metallocene-type catalysts; and J. Boor Jr., Ziegler-Natta Catalysts and Polymerizations, Academic Press, New York, 1979 for information about Ziegler-Natta-type catalysts.

Many of the useful polymerization conditions for these types of catalysts and the oligomerization catalyst coincide, so conditions for the process are easily accessible. Oftentimes a "cocatalyst" or "activator" is needed for metallocene or Ziegler-Natta-type polymerizations, much as W is sometimes needed for the oligomerization catalyst. In many instances the same compound, such as an alkylaluminum compound, may be used for these purposes for both types of catalysts.

Suitable catalysts for the copolymerization catalyst also include metallocene-type catalysts, as described in US5324800 and EP-A-0129368; particularly advantageous are bridged bis-indenyl metallocenes, for instance as described in US5145819 and EP-A-0485823. Another class of suitable catalysts comprises the well-known constrained geometry catalysts, as described in EP-A-0416815, EP-A-0420436, EP-A-0671404, EP-A-0643066 WO91/04257. Also the class of transition metal complexes described in, for example, WO98/30609, US5880241, US6060569 and US5714556 can be used. Metallocene catalysts already known for the copolymerization of active nonconjugated dienes are described in US5229478, WO88/04674, WO99/18135 and WO99/01460, and references described therein.

All the catalysts herein may be "heterogenized" (to form a polymerization catalyst component, for instance) by coating or otherwise attaching them to solid supports, such as silica or alumina. Where an active catalyst species is formed by reaction with a compound such as an alkylaluminum compound, a support on which the alkylaluminum compound is first coated or otherwise attached is contacted with the transition metal compounds (or their precursors) to form a catalyst system in which the active polymerization catalysts are "attached" to the solid support. These supported catalysts may be used in polymerizations in organic liquids. They may also be used in so-called gas phase polymerizations in which the olefin(s) being polymerized are added to the polymerization as gases and no liquid supporting phase is present. The transition metal compounds may also be coated onto a support such as a polyolefin (polyethylene, polypropylene, etc.) support, optionally along with other needed catalyst components such as one or more alkylaluminum compounds.

The oligomers made by the oligomerization catalyst and the polymer made by the polymerization catalyst may be made in sequence, i.e., the oligomerization followed by the polymerization, as by using two vessels in series. For example, ethylene can be oligomerized in a first reactor in the presence of the oligomerization catalyst to produce an oligomer mixture, which is then transferred to a second reactor with nonconjugated diene (to the extent not already present in the first monomer mixture) and additional ethylene/α-olefin feed (to the extent necessary), and polymerization catalyst, in the amounts and under polymerization conditions required for the desired end product.

However it is preferred to carry out the entire process in the same vessel(s), i.e., carrying out steps (1) and (2) sequentially or simultaneously. This is possible because in most instances the oligomerzation/polymerization catalysts and conditions are compatible with each other.

One such preferred process is to contact ethylene and the oligomerization catalyst for a period of time sufficient to oligomerize a portion of the ethylene to α-olefins, after which the copolymerization catalyst is added to the vessel. The nonconjugated diene, additional ethylene as needed, and other α-olefins as desired, can be added at any stage during the process.

Another preferred process is to add all components to the vessel at the same time - ethylene, nonconjugated diene, oligomerization catalyst and copolymerization catalyst - and conduct the oligomerization/copolymerization simultaneously. In this case, the amount of branching due to incorporation of the olefin H₂C=CHR¹⁸ in the polymer can be controlled by the ratio of oligomerization catalyst to copolymerization catalyst (not counting the nonconjugated diene). The higher the proportion of oligomerization catalyst the higher the amount of branching.

In all of these processes, it preferred to use essentially only ethylene and the active nonconjugated diene as monomers added into the process. Of course, other monomers/oligomers will be generated in situ and incorporated into the final copolymer, but the only monomers required to operate the process and generate the products are ethylene and the nonconjugated diene.

Preferably the amount of branching in the polyolefins formed by the process of the present invention is sufficient so that an elastomer is formed. By an elastomer is meant a polymer that has no melting point above 20°C whose heat of melting is 5 J/g or less, preferably 1 J/g or less (total if more than one melting point present), when measured by DSC at a heating rate of 10°C/min. The melting point is taken as the peak of the melting transition, and is taken on the second heat. Another preferred polymer is a semicrystalline polymer having a lower melting point than high density polyethylene, preferably a melting point lower than about 120°C, more preferably a melting point less than about 100°C.

A particularly preferred aspect of the process utilizes ethylene and the nonconjugated diene as the sole added monomers, with α-olefins being incorporated into the final copolymer solely as a result of the in situ oligomerization of ethylene.

In the Examples, all pressures are gauge pressures.

In the Examples the transition metal catalysts were either bought, or if a vendor is not listed, were made.

In the Examples, the following transition metal compounds are used.

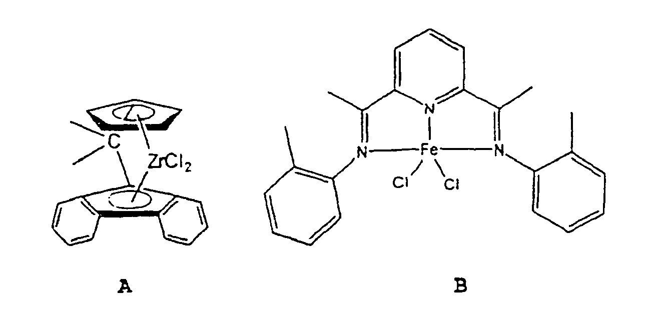

In the Examples, the following abbreviations are used:
MAO - methylaluminoxane
RT - room temperature

### Example 1

A 600mL Parr® reactor was cleaned, heated under vacuum and then allowed to cool under nitrogen. It was then brought into a drybox. In the drybox, to a Hoke® cylinder was added 5 mL toluene and 4.2 mL MAO (13.5 wt% toluene solution). To a 20 mL vial was added 2.0 mg A and 2 mL toluene. It was then pipet transferred to the 600 mL autoclave. Then 433 mg 0.1 wt% B in biphenyl was also added to the autoclave, followed by addition of 30 mL 5-ethylidene-2-norbornene and 120 mL 2,2,4-trimethylpentane. The autoclave was sealed. Both the Hoke® cylinder and the autoclave were brought out of the drybox. The autoclave was assembled to a high-pressure line. The Hoke® cylinder was then connected to the autoclave. The reactor was pressured with nitrogen and then the nitrogen pressure was released. The reactor was heated to 65°C, then pressurized 2X to 690 kPa ethylene, venting each time and finally pressurized to 820 kPa with stirring. The MAO solution was added from the Hoke® cylinder at slightly higher pressure. The ethylene pressure of the reactor was then adjusted to 1.24 MPa. The reaction mixture was allowed to stir around 90°C for 2h. The heat source was removed. Ethylene was vented to about 210 kPa. The reactor was back filled with 1.38 MPa nitrogen and was then vented to 210 kPa. This was repeated once. The reaction mixture was cooled to RT. It was then slowly poured into 400 mL methanol, followed by addition of 6 mL conc. HCl. After stirring at RT for 25 min, the polymer was filtered, washed with methanol six times and dried in vacuo. White powdery polymer (3.06 g) was obtained.

### Example 2

A 600 mL Parr® reactor was cleaned, heated under vacuum and then allowed to cool under nitrogen. It was then brought into a drybox. In the drybox, to a Hoke® cylinder was added 5 mL toluene and 4.2 mL MAO (13.5 wt% toluene solution). To a 20 mL vial was added 2.0 mg A and 2 mL toluene. It was then pipet transferred to the 600 mL reactor. Then 433 mg of 0.1 wt% B in biphenyl mixture was also added to the reactor, followed by addition of 20 mL 1,4-hexadiene and 130mL 2,2,4-trimethylpentane. The reactor was sealed. Both the Hoke® cylinder and the autoclave were brought out of the drybox. The autoclave was assembled to a high-pressure line. The Hoke® cylinder was connected to the autoclave. The reactor was pressured with nitrogen, and the nitrogen was then released. Reactor was heated to 65°C, then, pressurized 2X to 690 kPa ethylene, venting each time and finally pressurized to 830 kPa with stirring. The MAO solution was added from the Hoke® cylinder at slightly higher pressure. The ethylene pressure of the reactor was then adjusted to 1.24 MPa. The reaction mixture was allowed to stir at around 90°C for 40 min. The heat source was removed. Ethylene was vented to about 210 kPa. The reactor was back filled with 1.38 MPa nitrogen and was then vented to 210 kPa. This was repeated once. The reaction mixture was then cooled to RT. The reaction mixture was then slowly poured into 400 mL methanol, followed by addition of 6 mL conc. HCl. After stirring at RT for 25 min, the polymer was filtered, washed with methanol six times and dried in vacuo. White polymer (33.35 g) was obtained.

## Claims

1. A process for preparing a branched polyolefin containing olefinic bonds, comprising the steps of:
(1) contacting an ethylene oligomerization catalyst and a first monomer component comprising ethylene, under conditions to oligomerize at least a portion of the ethylene to one or more α-olefins; and
(2) contacting an active transition metal copolymerization catalyst, with a second monomer component comprising ethylene and at least a portion of the α-olefins from step (1), under conditions to copolymerize the second monomer component to a branched polyolefin,
**characterized in that** the ethylene oligomerization catalyst comprises an active Fe complex of a ligand of the formula (I) : 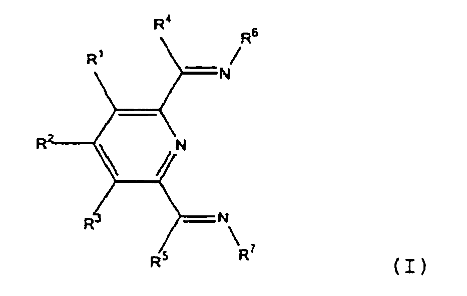 wherein:
R¹, R², R³, R⁴ and R⁵ are each independently hydrogen, hydrocarbyl, substituted hydrocarbyl, or an inert functional group, provided that any two of R¹, R² and R³ vicinal to one another, taken together may form a ring; and
R⁶ and R⁷ are aryl or substituted aryl; and
the second monomer component further comprises an active nonconjugated diene so that a branched polyolefin containing olefinic bonds is produced.

2. The process as recited in claim 1, **characterized in that** the oligomerization catalyst is an Fe complex of a ligand of the general formula (I), wherein:
R¹, R² and R³ are each independently hydrogen, hydrocarbyl, substituted hydrocarbyl or an inert functional group, provided that any two of R¹, R² and R³ vicinal to one another taken together may form a ring;
R⁴ and R⁵ are each independently hydrogen, hydrocarbyl, substituted hydrocarbyl or an inert functional group; R⁶ and R⁷ are each independently an aryl orsubstituted aryl having a first ring atom bound to the imino nitrogen, provided that:
in R⁶, a second ring atom adjacent to said first ring atom is bound to a halogen, a primary carbon group, a secondary carbon group or a tertiary carbon group; and further provided that
in R⁶, when said second ring atom is bound to a halogen or a primary carbon group, none, one or two of the other ring atoms in R⁶ and R⁷ adjacent to said first ring atom are bound to a halogen or a primary carbon group, with the remainder of the ring atoms adjacent to said first ring atom being bound to a hydrogen atom; or
in R⁶, when said second ring atom is bound to a secondary carbon group, none, one or two of the other ring atoms in R⁶ and R⁷ adjacent to said first ring atom are bound to a halogen, a primary carbon group or a secondary carbon group, with the remainder of the ring atoms adjacent to said first ring atom being bound to a hydrogen atom; or
in R⁶, when said second ring atom is bound to a tertiary carbon group, none or one of the other ring atoms in R⁶ and R⁷ adjacent to said first ring atom are bound to a tertiary carbon group, with the remainder of
the ring atoms adjacent to said first ring atom being bound to a hydrogen atom.

3. The process as recited in claim 2, **characterized in that** the oligomerization catalyst comprises an active Fe complex of a ligand of the formula (II): 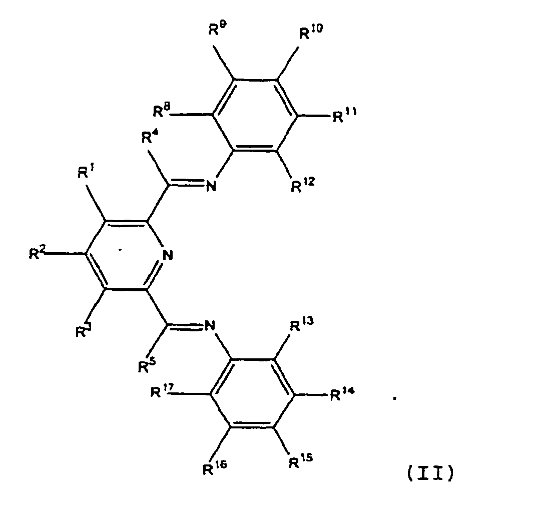 wherein:
each of R¹, R², R³, R⁴, R⁵, R⁹, R¹⁰, R¹¹, R¹⁴, R¹⁵ and R¹⁶ is independently selected from the group consisting of hydrogen, hydrocarbyl, substituted hydrocarbyl and an inert functional group; and
R⁸ is a primary carbon group, a secondary carbon group or a tertiary carbon group;
provided that:
when R⁸ is a primary carbon group none, one ortwo of R¹², R¹³ and R¹⁷ are independently a primary carbon group, an inert functional group or a trihalo tertiary carbon group, and the remainder of R¹², R¹³ and R¹⁷ are hydrogen;
when R⁸ is a secondary carbon group, none or one of R¹², R¹³ and R¹⁷ is a primary carbon group, a secondary carbon group, a trihalo tertiary carbon group or an inert functional group, and the remainder of R¹², R¹³ and R¹⁷ are hydrogen;
when R⁸ is a tertiary carbon group all of R¹², R¹³ and R¹⁷ are hydrogen;
any two of R¹, R² and R³ vicinal to one another, taken together may form a ring.; and
any two of R⁸, R⁹, R¹⁰, R¹¹, R¹², R¹³, R¹⁴, R¹⁵, R¹⁶ and R¹⁷ vicinal to one another, taken together may form a ring.

4. The process as recited in claim 1, **characterized in that** said active nonconjugated diene is selected from the group consisting of 1,4-hexadiene, ethylidene norbornene and dicyclopentadiene.

5. The process as recited in claim 1, **characterized in that** steps (1) and (2) are conducted sequentially or simultaneously in the same reactor vessel.

6. The process as recited in claim 5, **characterized in that** steps (1) and (2) are conducted simultaneously in the same reactor vessel.

7. The process as recited in claim 6, **characterized in that** the oligomerization and copolymerization occur at comparable rates.

8. The process as recited in claim 1, **characterized in that** the first monomer component consists essentially of ethylene and optionally the active nonconjugated diene, and the second monomer component consists essentially of ethylene, at least a portion of the α-olefins from step (1) and the active nonconjugated diene.

9. The process as recited in claim 1, **characterized in that** one or more of the catalysts is supported.

10. A branched polyolefin containing at least 2 ethyl branches, at least 2 hexyl or longer branches and at least one butyl branch per 1000 methylene groups, **characterized in that** said branched polyolefin further contains olefinic bonds.

11. The branched polyolefin as recited in claim 10, **characterized in that** said olefinic bonds are contained in repeat units derived from an active nonconjugated diene.

12. The branched polyolefin as recited in claim 11, **characterized in that** it is elastomeric.

13. A branched polyolefin, containing 20 to 150 branches of the formula -(CH₂CH₂)ₙH wherein n is an integer of 1 to 100, **characterized in that** said branched polyolefin further contains olefinic bonds.

14. The branched polyolefin as recited in claim 13, **characterized in that** said olefinic bonds are contained in repeat units derived from an active nonconjugated diene.

15. The branched polyolefin as recited in claim 14, **characterized in that** it is elastomeric.

## Patentansprüche

1. Verfahren zum Herstellen eines verzweigten Polyolefins, enthaltend olefinische Bindungen, die Stufen umfassend von:
(1) in Kontakt bringen einen Ethylen-Oligomerisierungskatalysator und eine erste Monomerkomponente, umfassend Ethylen, unter Bedingungen um mindestens einen Teil des Ethylens an eines oder mehrere α-Olefine zu oligomerisieren, und
(2) in Kontakt bringen einen aktiven Übergangsmetallcopolymerisationskatalysator mit einer zweiten Monomerkomponente, umfassend Ethylen und mindestens einen Teil der α-Olefine aus Stufe (1), unter Bedingungen, die zweite Monomerkomponente zu einem verzweigten Polyolefin zu copolymerisieren,
**dadurch gekennzeichnet, daß** der Ethylen Oligomerisierungskatalysator umfaßt einen aktiven Fe Komplex eines Liganden der Formel (I): 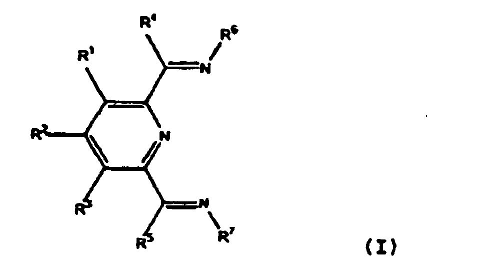 wobei R¹, R², R³, R⁴ und R⁵ jeweils unabhängig Wasserstoff, Hydrocarbyl, substituiertes Hydrocarbyl, oder eine inerte funktionelle Gruppe sind, vorausgesetzt, daß irgendwelche zwei von R¹, R² und R³, benachbart zueinander, zusammengenommen einen Ring bilden können, und
R⁶ und R⁷ sind Aryl oder substituiertes Aryl; und die zweite Monomerkomponente umfaßt ferner ein aktives nicht konjugiertes Dien, so daß ein verzweigtes Polyolefin, enthaltend olefinische Bindungen, hergestellt wird.

2. Verfahren nach Anspruch 1, **dadurch gekennzeichnet, daß** der Oligomerisierungskatalysator ein Fe Komplex eines Liganden der allgemeinen Formel (I) ist, wobei
R¹, R² und R³ jeweils unabhängig Wasserstoff, Hydrocarbyl, substituiertes Hydrocarbyl oder eine inerte funktionelle Gruppe sind, vorausgesetzt, daß irgendwelche zwei von R¹, R² und R³, benachbart zueinander, zusammen genommen einen Ring bilden können,
R⁴ und R⁵ sind jeweils unabhängig Wasserstoff, Hydrocarbyl, substituiertes Hydrocarbyl oder eine inerte funktionelle Gruppe,
R⁶ und R⁷ sind jeweils unabhängig ein Aryl oder substituiertes Aryl mit einem ersten Ringatom, gebunden an den Iminostickstoff, vorausgesetzt daß:
in R⁶ ein zweites Ringatom benachbart zu dem ersten Ringatom an ein Halogen, eine primäre Kohlenstoffgruppe, eine sekundäre Kohlenstoffgruppe oder eine tertiäre Kohlenstoffgruppe gebunden ist, und ferner unter der Voraussetzung, daß
in R⁶, wenn das zweite Ringatom an ein Halogen oder eine primäre Kohlenstoffgruppe gebunden ist, keines, eines oder zwei der anderen Ringatome in R⁶ und R⁷, benachbart zu dem ersten Ringatom, an ein Halogen oder eine primäre Kohlenstoffgruppe gebunden sind, wobei der Rest der Ringatome, benachbart zu dem ersten Ringatom, an ein Wasserstoffatom gebunden ist, oder
in R⁶, wenn das zweite Ringatom an eine sekundäre Kohlenstoffgruppe gebunden ist, ist keines, eines oder zwei der anderen Ringatome in R⁶ und R⁷, benachbart zu dem ersten Ringatom, an ein Halogen, eine primäre Kohlenstoffgruppe oder eine sekundäre Kohlenstoffgruppe gebunden, wobei der Rest der Ringatome, benachbart zu dem ersten Ringatom, an ein Wasserstoffatom gebunden ist, oder
in R⁶, wenn das zweite Ringatom an eine tertiäre Kohlenstoffgruppe gebunden ist, ist keines oder eines der anderen Ringatome in R⁶ und R⁷, benachbart zu dem ersten Ringatom, an eine tertiäre Kohlenstoffgruppe gebunden, wobei der Rest der Ringatome, benachbart zu dem ersten Ringatom, an ein Wasserstoffatom gebunden ist.

3. Verfahren nach Anspruch 2, **dadurch gekennzeichnet, daß** der Oligomerisierungskatalysator einen aktiven Fe Komplex eines Liganden der Formel (II) umfaßt: 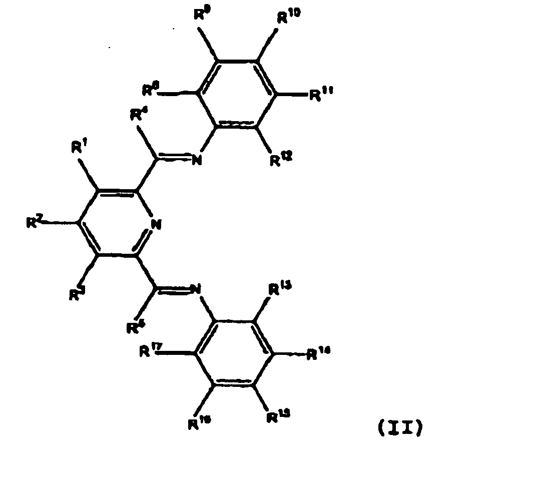 wobei:
jedes R¹, R², R³, R⁴, R⁵, R⁹, R¹⁰, R¹¹, R¹⁴, R¹⁵ und R¹⁶ unabhängig ausgewählt ist aus der Gruppe, bestehend aus Wasserstoff, Hydrocarbyl, substituiertes Hydrocarbyl und einer inerten funktionellen Gruppe, und
R⁸ ist eine primäre Kohlenstoffgruppe, eine sekundäre Kohlenstoffgruppe oder eine tertiäre Kohlenstoffgruppe, vorausgesetzt, daß:
wenn R⁸ eine primäre Kohlenstoffgruppe ist, keines, eines oder zwei von R¹², R¹³ und R¹⁷ unabhängig eine primäre Kohlenstoffgruppe, eine inerte funktionelle Gruppe oder eine trihalogene tertiäre Kohlenstoffgruppe sind, und der Rest von R¹², R¹³ und R¹⁷ ist Wasserstoff,
wenn R⁸ eine sekundäre Kohlenstoffgruppe ist, ist keines oder eines von R¹², R¹³ und R¹⁷ eine primäre Kohlenstoffgrupe, eine sekundäre Kohlenstoffgruppe, eine trihalogene tertiäre Kohlenstoffgruppe oder eine inerte funktionelle Gruppe, und der Rest von R¹², R¹³ und R¹⁷ ist Wasserstoff,
wenn R⁸ eine tertiäre Kohlenstoffgruppe ist, sind alle von R¹², R¹³ und R¹⁷ Wasserstoff,
irgendwelche zwei von R¹, R² und R³, benachbart zueinander, können zusammengenommen einen Ring bilden, und
irgendwelche zwei von R⁸, R⁹, R¹⁰, R¹¹, R¹², R¹³, R¹⁴, R¹⁵, R¹⁶ und R¹⁷, benachbart zueinander, können zusammen genommen einen Ring bilden.

4. Verfahren nach Anspruch 1, **dadurch gekennzeichnet, daß** das aktive nicht konjugierte Dien ausgewählt wird aus der Gruppe, bestehend aus 1,4-Hexadien, Ethyliden, Norbornen und Dicyclopentadien.

5. Verfahren nach Anspruch 1, **dadurch gekennzeichnet, daß** Stufen (1) und (2) aufeinanderfolgend oder gleichzeitig in dem gleichen Reaktorkessel durchgeführt werden.

6. Verfahren nach Anspruch 5, **dadurch gekennzeichnet, daß** Stufen (1) und (2) gleichzeitig in dem gleichen Reaktorkessel durchgeführt werden.

7. Verfahren nach Anspruch 6, **dadurch gekennzeichnet, daß** die Oligomerisierung und Copolymerisation mit vergleichbaren Raten auftreten.

8. Verfahren nach Anspruch 1, **dadurch gekennzeichnet, daß** die erste Monomerkomponente im wesentlichen aus Ethylen und wahlfrei dem aktiven nicht konjugiertem Dien besteht, und die zweite Monomerkomponente besteht im wesentlichen aus Ethylen, mindestens einem Teil der α-Olefine aus Stufe (1) und dem aktiven nicht konjugiertem Dien.

9. Verfahren nach Anspruch 1, **dadurch gekennzeichnet, daß** einer oder mehrere der Katalysatoren unterstützt ist.

10. Verzweigtes Olefin, enthaltend mindestens 2 Ethylzweige, mindestens zwei Hexyl- oder längere Zweige und mindestens einen Butylzweig pro 1000 Methylengruppen, **dadurch gekennzeichnet, daß** das verzweigte Polyolefin ferner olefinische Bindungen enthält.

11. Verzweigtes Polyolefin nach Anspruch 10, **dadurch gekennzeichnet, daß** die olefinischen Bindungen in Wiederholungseinheiten, abstammend von einem aktiven nichtkonjugiertem Dien, enthalten sind.

12. Verzweigtes Polyolefin nach Anspruch 11, **dadurch gekennzeichnet, daß** es elastomer ist.

13. Verzweigtes Polyolefin, enthaltend 20 bis 150 Zweige der Formel -(CH₂CH₂)ₙH, wobei n eine ganze Zahl von 1 bis 100 ist, **dadurch gekennzeichnet, daß** das verzweigte Polyolefin ferner olefinische Bindungen enthält.

14. Verzweigtes Polyolefin nach Anspruch 13, **dadurch gekennzeichnet, daß** die olefinischen Bindungen in Wiederholungseinheiten, abstammend von einem aktiven nicht konjugierten Dien enthalten, sind.

15. Verzweigtes Polyolefin nach Anspruch 14, **dadurch gekennzeichnet, daß** es elastomer ist.

## Revendications

1. Un processus de préparation d'une polyoléfine ramifiée qui contient des liaisons oléfiniques, comprenant les étapes suivantes:
(1) la mise en contact d'un catalyseur d'oligomérisation de l'éthylène et d'un premier composant monomère comportant de l'éthylène dans des conditions qui assurent l'oligomérisation d'au moins une portion de l'éthylène en une ou plusieurs α-oléfines; et
(2) la mise en contact d'un catalyseur de copolymérisation contenant un métal de transition actif avec un second composant monomère comportant de l'éthylène et une portion au moins des α-oléfines obtenues à l'étape (1) dans des conditions qui assurent la copolymérisation du second composant monomère à une polyoléfine ramifiée,
**caractérisé par le fait que** le catalyseur d'oligomérisation de l'éthylène comprend un complexe Fe actif d'un ligand de formule (I): 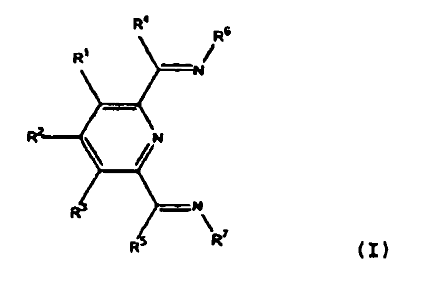 où:
R¹, R², R³, R⁴ et R⁵ représentent chacun indépendamment un atome d'hydrogène, un groupement hydrocarbyle, un groupement hydrocarbyle substitué ou un groupement fonctionnel inerte, à condition que deux quelconques des R¹, R² et R³ vicinaux l'un de l'autre pris ensemble puissent former un cycle; et
R⁶ et R⁷ représentent un groupement aryle ou un groupement aryle substitué; et le second composant monomère comprend également un diène actif non conjugué, de façon à ce qu'une polyoléfine ramifiée contenant des liaisons oléfiniques soit produite.

2. Le processus selon la revendication 1, **caractérisé par le fait que** le catalyseur d'oligomérisation est un complexe Fe d'un ligand de formule générale (I) dans laquelle:
R¹, R² et R³ représentent chacun indépendamment un atome d'hydrogène, un groupement hydrocarbyle, un groupement hydrocarbyle substitué ou un groupement fonctionnel inerte, à condition que deux quelconques des R¹, R² et R³ vicinaux l'un de l'autre pris ensemble puissent former un cycle;
R⁴ et R⁵ représentent chacun indépendamment un atome d'hydrogène, un groupement hydrocarbyle, un groupement hydrocarbyle substitué ou un groupement fonctionnel inerte;
R⁶ et R⁷ représentent chacun indépendamment un groupement aryle ou un groupement aryle substitué dont un premier atome du cycle est lié à l'azote du groupement imino, à condition que:
dans R⁶, un second atome du cycle adjacent audit premier atome du cycle est lié à un atome d'halogène, un groupement carboné primaire, un groupement carboné secondaire ou un groupement carboné tertiaire; et à condition également que
dans R⁶, quand ledit second atome du cycle est lié à un atome d'halogène ou à un groupement carboné primaire, zéro, un ou deux des autres atomes du cycle dans R⁶ et R⁷ adjacents audit premier atome du cycle sont liés à un atome d'halogène ou à un groupement carboné primaire, les atomes restants du cycle adjacents audit premier atome du cycle étant liés à un atome d'hydrogène; ou
dans R⁶, quand ledit second atome du cycle est lié à un groupement carboné secondaire, zéro, un ou deux des autres atomes du cycle dans R⁶ et R⁷ adjacents audit premier atome du cycle sont liés à un atome d'halogène, à un groupement carboné primaire ou à un groupement carboné secondaire, les atomes restants du cycle adjacents audit premier atome du cycle étant liés à un atome d'hydrogène; ou
dans R⁶, quand ledit second atome du cycle est lié à un groupement carboné tertiaire, zéro ou un des autres atomes du cycle dans R⁶ et R⁷ adjacents audit premier atome du cycle est lié à un groupement carboné tertiaire, les atomes restants du cycle adjacents audit premier atome du cycle étant liés à un atome d'hydrogène.

3. Le processus selon la revendication 2, **caractérisé par le fait que** le catalyseur d'oligomérisation comprend un complexe Fe d'un ligand de formule (II): 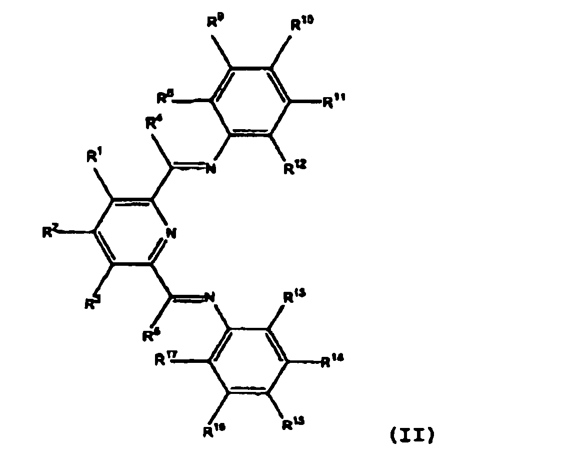 où:
R¹, R², R³, R⁴, R⁵, R⁹, R¹⁰, R¹¹, R¹⁴, R¹⁵ et R¹⁶ sont chacun indépendamment sélectionnés parmi le groupe consistant en un atome d'hydrogène, un groupement hydrocarbyle, un groupement hydrocarbyle substitué et un groupement fonctionnel inerte; et
R⁸ représente un groupement carboné primaire, un groupement carboné secondaire ou un groupement carboné tertiaire; à condition que:
quand R⁸ représente un groupement carboné primaire, zéro, un ou deux des R¹², R¹³ et R¹⁷ représentent indépendamment un groupement carboné primaire, un groupement fonctionnel inerte ou un groupement carboné tertiaire trihalogéné et les R¹², R¹³ et R¹⁷ restants représentent un atome d'hydrogène;
quand R⁸ représente un groupement carboné secondaire, zéro ou un des R¹², R¹³ et R¹⁷ représentent un groupement carboné primaire, un groupement carboné secondaire, un groupement carboné tertiaire trihalogéné ou un groupement fonctionnel inerte et les R¹², R¹³ et R¹⁷ restants représentent un atome d'hydrogène;
quand R⁸ représente un groupement carboné tertiaire, R¹², R¹³ et R¹⁷ représentent tous un atome d'hydrogène;
deux quelconques des R¹, R² et R³ vicinaux l'un de l'autre pris ensemble peuvent former un cycle; et
deux quelconques des R⁸, R⁹, R¹⁰, R¹¹, R¹², R¹³, R¹⁴, R¹⁵, R¹⁶ et R¹⁷ vicinaux l'un de l'autre pris ensemble peuvent former un cycle.

4. Le processus selon la revendication 1, **caractérisé par le fait que** ledit diène actif non conjugué est sélectionné parmi le groupe consistant en le 1,4-hexadiène, l'éthylidène norbornène et le dicyclopentadiène.

5. Le processus selon la revendication 1, **caractérisé par le fait que** les étapes (1) et (2) sont réalisées en succession ou simultanément dans la même cuve de réaction.

6. Le processus selon la revendication 5, **caractérisé par le fait que** les étapes (1) et (2) sont réalisées simultanément dans la même cuve de réaction.

7. Le processus selon la revendication 6, **caractérisé par le fait que** l'oligomérisation et la copolymérisation ont lieu à des vitesses comparables.

8. Le processus selon la revendication 1, **caractérisé par le fait que** le premier composant monomère consiste essentiellement en de l'éthylène et en option le diène actif non conjugué, et le second composant monomère consiste essentiellement en de l'éthylène, une portion au moins des α-oléfines obtenues à l'étape (1) et le diène actif non conjugué.

9. Le processus selon la revendication 1, **caractérisé par le fait que** un ou plusieurs des catalyseurs sont soutenus.

10. Une polyoléfine ramifiée contenant au moins deux chaînes éthyles, au moins deux chaînes hexyles ou plus longues et au moins une chaîne butyle par 1 000 groupements méthylène, **caractérisée par le fait que** ladite polyoléfine ramifiée contient également des liaisons oléfiniques.

11. La polyoléfine ramifiée selon la revendication 10, **caractérisée par le fait que** lesdites liaisons oléfiniques sont contenues dans des unités répétées dérivées d'un diène actif non conjugué.

12. La polyoléfine ramifiée selon la revendication 11, **caractérisée par le fait qu'**elle est élastomère.

13. Une polyoléfine ramifiée comportant de 20 à 150 chaînes de formule -(CH₂CH₂)ₙH où n est un nombre entier compris entre 1 et 100, **caractérisée par le fait que** ladite polyoléfine ramifiée contient également des liaisons oléfiniques.

14. La polyoléfine ramifiée selon la revendication 13, **caractérisée par le fait que** lesdites liaisons oléfiniques sont contenues dans des unités répétées dérivées d'un diène actif non conjugué.

15. La polyoléfine ramifiée selon la revendication 14, **caractérisée par le fait qu'**elle est élastomère.
